# EUROPEAN PATENT APPLICATION

(11) **EP 1 130 028 A1**
(43) Date of publication of application: **05.09.2001**
(21) Application number: 00830118.6
(22) Date of filing: 18.02.2000
(51) Int. Cl.: C07K 14/47, C07K 14/52

(54) **Process for the preparation of a mucosal form of the eosinophil cationic protein (ECP), mucosal ECP and uses thereof as allergic phlogosis marker**

(71) Applicant: Marcucci, Francesco, I-06100 Perugia (IT); Frati, Franco, 52044 Cortona (Ar) (IT)
(72) Inventor: Marcucci, Francesco, I-06100 Perugia (IT); Frati, Franco, 52044 Cortona (Ar) (IT)
(74) Representative: Capasso, Olga

(57) **Abstract**

It is described a process for the preparation of ECP from nasal mucosa of a subject; the ECP protein so obtainable; and a method for monitoring the phlogosis from allergic reaction of a subject.

## Description

The present invention relates to a process for the preparation of a mucosal form of the Eosinophil Cationic Protein (ECP) as allergic phlogosis marker, to be used in a method for the detection of ECP.

More particularly the invention relates to a process for the purification of the ECP which includes the step of taking and isolating ECP from the place where it exerts its pathogenic effects, as well as from subjects where it is present in a functional form, i.e. during the course of the process responsible of the disease. Presently the ECP used for detection methods is obtained from blood of healthy donors.

### Prior Art

The ECP protein (eosinophil cationic protein) is used as marker for monitoring of inflammatory processes (1, 2) which result in a number of allergic diseases, like asthma and rhinitis (3, 4, 5).

Up to now the monitoring methods for allergic phlogosis are carried out by means of nasal washing liquids obtained from patients affected by allergic rhinitis (6, 7), the sputum (8, 9) and serum from patients affected by asthma (10, 11). Appreciable results are obtained but sometimes they are contrasting and not completely convincing in order to carry out clinical routine use thereof (9, 11, 12, 13).

Partially unsuccessful results obtained from the use of ECP as phlogosis marker can be ascribed to the utilisation of ECP, as antigen for the production of antibodies subsequently used in the detection assays, in a functional and structural form not corresponding to that obtained during the phlogosis process, this last being the one which is substantially detected in the target organ.

ECP used as antigen for the production of antigens up to now has been extracted and purified from the serum of healthy or eosinophilia affected subjects (14), and mainly from eosinophils contained in the peripheral blood of healthy donors (15).

ECP is a quite heterogeneous protein having a molecular weight in the range from 16.000 to 22.000 KDa (16, 17), and exerting numerous and different functions in the various organic districts and different clinical conditions (18, 19). It is recognised that ECP exhibits cytotoxic activity on parasites and tumour cells, neurotoxicity, potential damages on cardiovascular system and respiratory epithelial cells, anti-bacterial and anti-viral activity. The capacity to inhibit the T cell proliferation, release of hystamine from basophils, activate the heart mast-cells, modify the production of proteoglycans from fibroblasts, stimulate the mucus secretion from aerial tracts, be able to shorten and lengthen coagulation times at low and high doses, respectively, pre-activate the plasminogen, inhibit the streptokinase, be antagonist for heparin, interact with the complement system, induce the expression of the IG17-1 receptor in bronchial epithelial cells, stimulate the expression of IACM-1 on epithelial cells, are described as some examples among EPC not toxic effects.

It is believed that these different activities are to be ascribed to the different functional forms in which ECP is produced by activated eosinophils, which are dependent on the membrane receptors, cytokines and mediators contained in the organic district wherein the phlogosis process occurs (18, 20, 21). Presently six different ECP forms are known and only one is capable, for example, to exert a specific cytoxic activity against erythrocytic leukemic cells (19).

ECP is transported out of the eosinophils substantially according two ways, i.e. by secretion of the activated cells (22), and by cytolysis of apoptotic cells present on mucosa surfaces of target organ (23, 24, 25, 26).

Although the activation process of the eosinophils can begin in the peripheral circulus, the processes of activation, secretion and cytolysis effectively occur in the microenvironment of the target organ, wherein these cells are massively collected (24, 26, 27, 28).

Accordingly ECP levels are much higher on mucosa surfaces of target organs than in serum of the same patients (28, 29). Furthermore ECP levels after its provocation by specific allergen significantly increase in the target organ rather than in the serum (28, 29). In addition free granules of eosinophils derived form cytolysis induced by specific provocation have been detected exclusively on the mucosa surfaces of aerial tracts (27, 28).

### Description of the Invention

The authors of the present invention provided a method for the production of a phlogosis marker in a more specific and sensitive form than up to now obtained by the extraction of ECP from peripheral circulus of healthy donors. Accordingly the authors provided a method for the preparation of a new ECP functional form, i.e. in the same form according to which it is produced in the target organ during the physiopathologic process whereby the disease occurs.

The method substantially includes:
1. Withdrawing of ECP directly from the mucosa surfaces of the organ wherein the phlogosis process is going on.
2. Obtaining ECP having a conformation and structure which is responsible for its pathogenic activity, because withdrawn during the pathologic event determined by the same.
3. Carrying out the withdrawing in such conditions to reproduce the pathogenic event according to a methodology consolidated by clinical practice including the specific provocation test by the use of the allergen responsible of allergic symptoms (test for nasal specific provocation, T.P.N.).
4. Withdrawing of biological material from the mucosa surfaces in its native conditions and without the use of washing liquids in order to obtain a concentration suitable for the subsequent protein extraction.
5. Providing a technique for the extraction and purification which allows to obtain considerable amounts of a new ECP form having the most suitable antigenic structure to monitor the pathologic process provoked by the same.

Therefore according to the method of the invention is provided a phlogosis marker, constituted by an ECP form other than that derived from serum as to electric charge and molecular weight and contained in high amount with respect to the volume of starting sample.

The provision of this ECP active form at high concentrations allows to achieve essentially the following advantages:
1. Obtaining a phlogosis marker more sensitive and specific to carry out the determination of ECP in biological liquids from mucosa surfaces because obtained from the same biological liquids wherein it is produced.
2. Obtaining an ECP having an activated form because obtained from mucosa surfaces of subjects in which the physiological event responsible of phlogosis occurs and therefore other than that obtained from blood of healthy donors.
3. Providing a more accurate method to monitor the allergic inflammation characterised by a better correlation with the symptoms induced by said phlogosis in the target organ.
4. Obtaining an ECP form using a method less damaging and debilitating and more economic than the donation of high amount of blood.

Therefore it is an object of the present invention a process for the preparation of ECP from nasal mucosa of a subject including the steps of:
- submitting the subject to a provocation by means of an allergen able to induce the allergic phlogosis;
- withdrawing after 24 hours from said subject nasal mucus and putting the same in the presence of a reducing agent;
- submitting said mucus to agitation and centrifugation in order to obtain a supernatant;
- dissolving in said supernatant a chelating agent and concentrating the same from 5 to 30 times to obtain a concentrated sample;
- submitting the concentrated sample to separation processes by molecular weight and/or ionic charge.

Preferably the allergen suitable to induce the allergic phlogosis is *Dermathophagoides pteronisinus* (ALK-Abello). Preferably the reducing agent is dithiothreitol. Preferably the chelating agent is EDTA. Preferably the processes of separation by molecular weight include gel-filtration on Sephadex G-75 column. Preferably the processes of separation by ionic charge include ion exchange chromatography on CM-cellulose column.

It is a further object of the present invention the ECP protein obtainable according to the method of the invention form nasal mucus of subjects provoked by allergen.

It is within the scope of the invention a method for monitoring phlogosis from allergic reaction of a subject including the steps of:
- incubating a biological fluid of the subject with an antibody obtained by immunization using the ECP of the invention;
- detecting the antigen-antibody reaction.

Preferably the detection of the antigen-antibody reaction is carried out by means of an additional antibody conjugated with a marker.

The present invention will be described in an exemplifying but non limiting way with reference to the following drawings:
Figure 1: histogram showing the amount of ECP obtained by nasal washings (L.N.) together with mucus expulsion (M.E.).
Figure 2: histogram showing the amount of serum and nasal ECP before (P.) and after (D.) T.P.N. treatment.
Figure 3: histogram showing the amount of serum and nasal ECP capable to bind EG2 antibody before (P.) and after (D.) T.P.N. treatment.
Figure 4: elution diagram for ECP protein from G-75 column.
Figure 5: elution diagram for ECP protein from CM-cellulose column.
Figures 6a and 6b: western blotting of native proteins form serum (a) and nasal washing (b) on PAGE 7.5% acrylamide using 1:1 diluted mAb1 antibody (Pharmacia).
Figure 7: western blotting of native proteins from serum (S.) and nasal washing (L.N.) on PAGE 7.5% acrylamide using EG1, EG2 and mAb1 (Pharmacia) antibodies. MAb1 and EG1 antibodies recognise the total ECP protein (active and inactive forms) whereas EG2 antibody recognises only the active form.
Figure 8: SDS-PAGE 12.5% acrylamide treatment of 42, 44, 46, 50 and 52 chromatographic fractions obtained after elution on CM-cellulose. M. = molecular weight markers.

### Materials and Methods

The study was carried out considering 16 out of 40 patients selected being affected by allergic asthma and rhinitis and not subjected to any therapy: ECP levels were determined in samples collected both by washing with physiological solution (10 ml) and from nasal mucus expulsed by patients directly in receptacles; samples were diluted 1:1 using dithiothreitol 0.2%, 2 mM TBS (about 1 ml for each sample).

Before and 24 hours after the nasal provocation using specific allergen (*Dermathophagoides pteronisinus* (ALK-Abello), the levels of ECP and activated ECP (detectable by EG2 antibody, Pharmacia) were determined in serum and nasal samples collected from 40 patients by direct expulsion of nasal mucus in the receptacles.

After agitation over 30 minutes at 4°C and centrifugation over 15 minutes at 3000 rpm, 50 ml of supernatant of nasal samples collected by direct expulsion 24 hours after the allergen provocation were dialysed against 2 mM EDTA in TBS and concentrated to 2.5 ml (20 times) by ultrafiltration using a Millipore YM3 membrane having a 62 mm diameter. ECP was determined before and after the concentration.

Concentrated sample was subjected to gel-filtration on Sephadex G-75 (2 x 85 cm) column eluting with 0.2 M ammonium acetate, pH 4, and the ECP content in the fractions eluted over 12 hours at 4°C was determined.

After dialysis and concentration by ultrafiltration of the ECP containing fractions the sample was subjected to a ion exchange chromatography on CM-cellulose column (2 x 20 cm) and the ECP content in the fractions eluted using 10 mM ammonium acetate buffer, pH 8.4, over 12 hours at 4°C, was determined.

After the determination of ECP content, the eluted fractions containing ECP finally were further concentrated by dialysis and ultrafiltration and frozen at -20°C,

The ECP determination was carried out using CAP SYSTEM FEIA available from Pharmacia Company and using also solid phases coupled with EG2 antibody also available from the same Company.

The ECP characterisation in the nasal samples and serum from a few patients was carried out by Western-Blotting after electrophoresis in native conditions on 7.5% acrylamide gel, using Mab1, EG1 and EG2 (Pharmacia) antibodies as primary antibodies and an antibody conjugated with alkaline phosphatase as secondary antibody.

After elution on Sephadex G-75 and CM-cellulose the fractions concentrated by ultrafiltration were subjected to 12.5% acrylamide gel SDS electrophoresis using protein markers having known molecular weight.

The molecular weight of nasal ECP protein was re-confirmed by repeating the gel-filtration on Sephadex G-75 column and using markers with different molecular weight for a SDS-PAGE on 12.5% acrylamide gel.

### Results

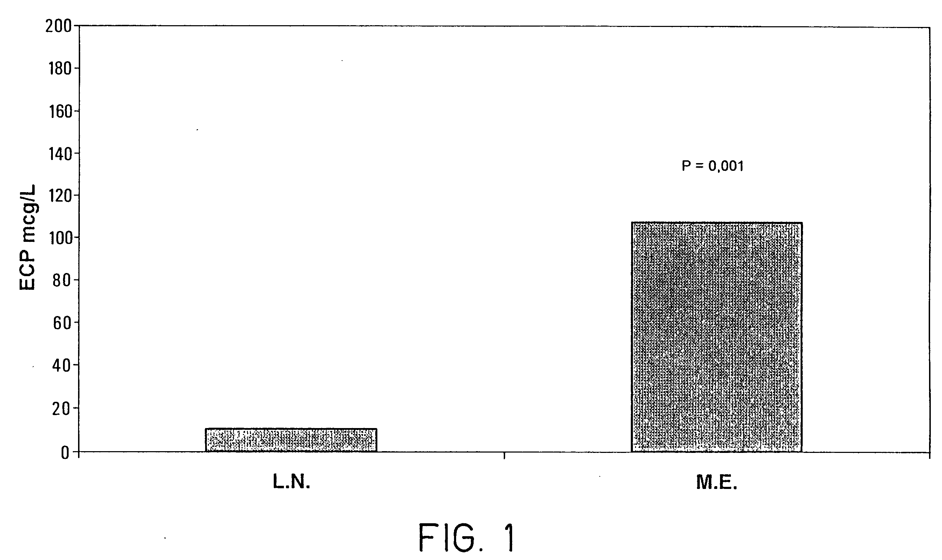

The samples obtained by direct expulsion of mucus on average show ECP values 20 times higher than samples obtained by nasal washing (Fig. 1); therefore this material was used for the collection.

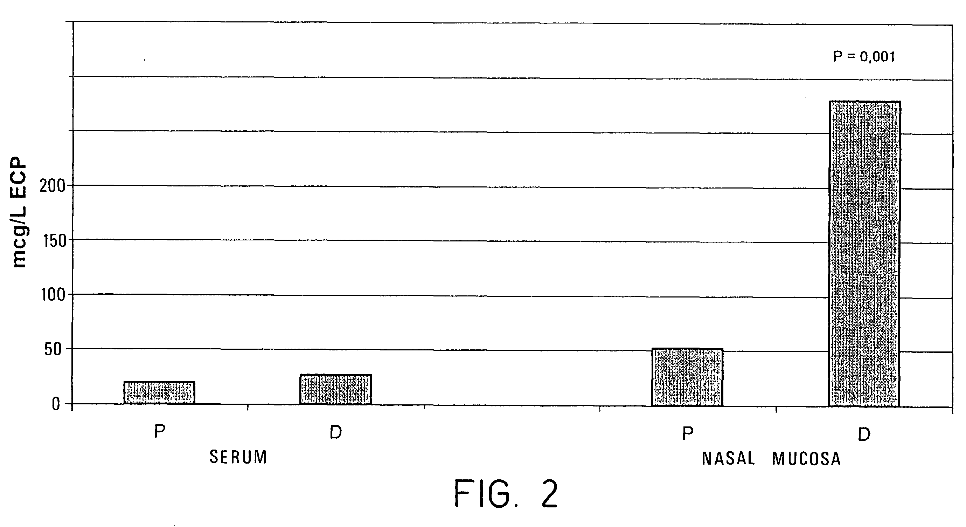

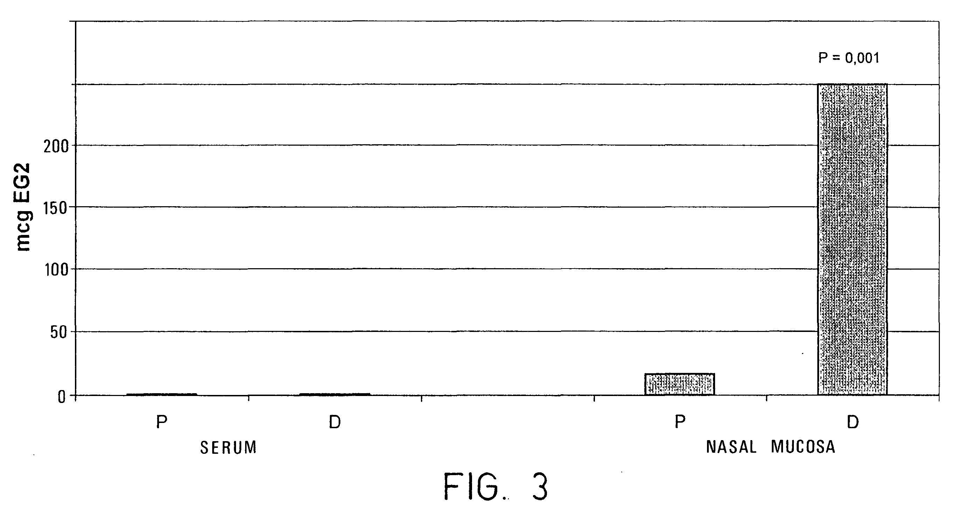

After the nasal provocation test using specific allergen the levels of nasal ECP are significantly higher than basal values (Fig. 2), particularly with respect to the content of ECP activated form detected by EG2 antibody, which was absent in the serum from the same patients (Fig. 3). Therefore for the extraction of the ECP protein it was decided to use nasal samples obtained 24 hours after T.P.N. allergen provocation.

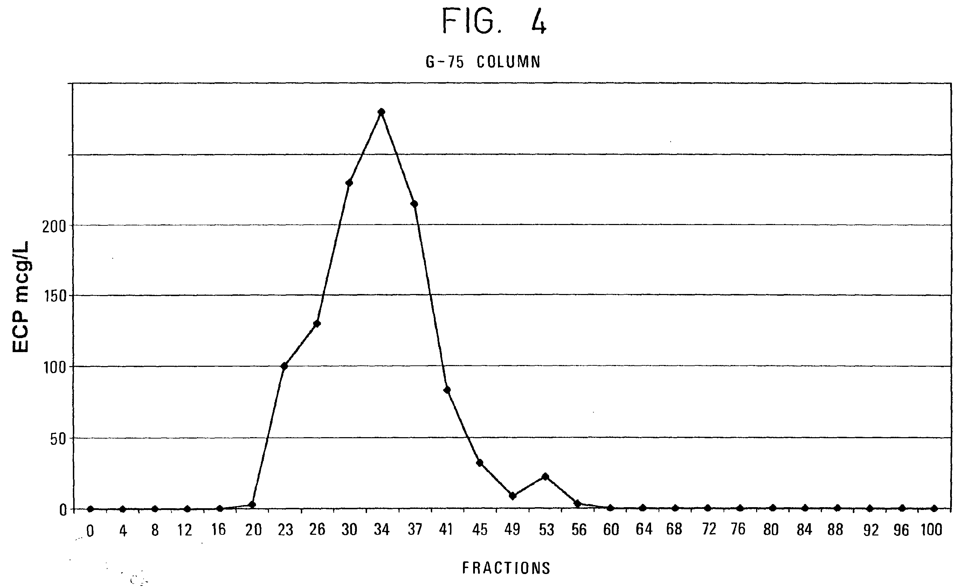

After dialysis and concentration by ultrafiltration to a 2.5 ml volume from a 50 ml starting volume, the gel filtration on Sephadex G-75 column distributes ECP in a sharp fraction peak (Fig. 4), which are further dialysed and concentrated by ultrafiltration to a 2.5 ml volume.

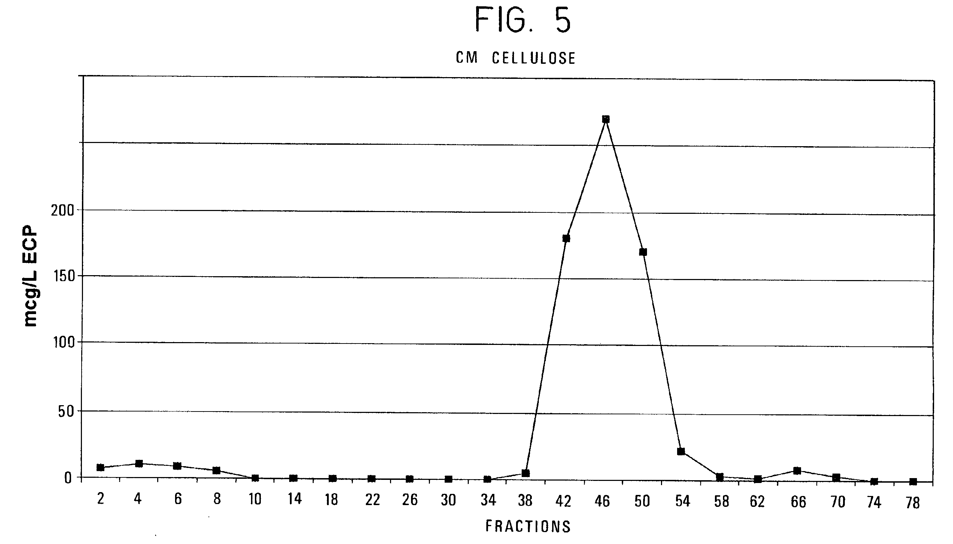

The next elution on a ion exchange CM-cellulose column shows that ECP is present at high level in a fraction peak (Fig. 5), which after further concentration to 2.5 ml volume show to contain a remarkable amount of ECP (about 1.2 mg/2.5 ml). The sample was stored at-20°C.

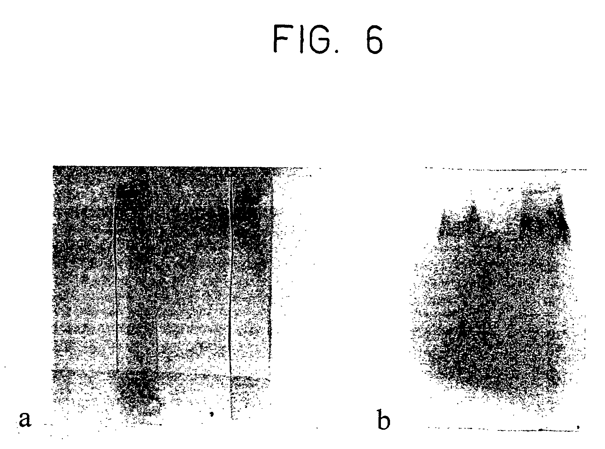

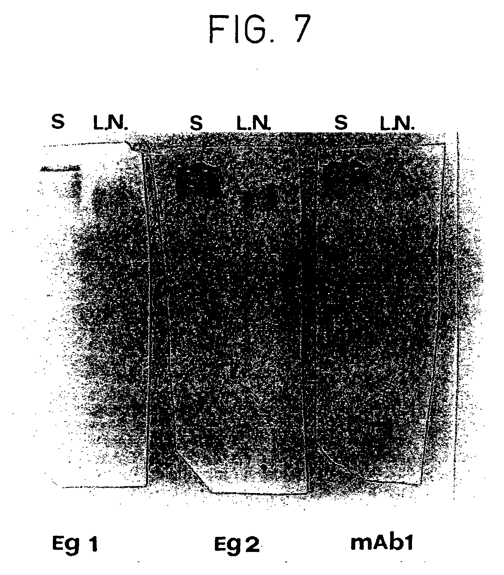

When nasal and serum samples of this protein, characterised by Western-blotting after electrophoresis in native conditions, were compared it appears that the nasal samples have lower electric charge than serum samples, likely due to the different glycosilation conditions (Fig. 6A, Fig. 6B, Fig. 7).

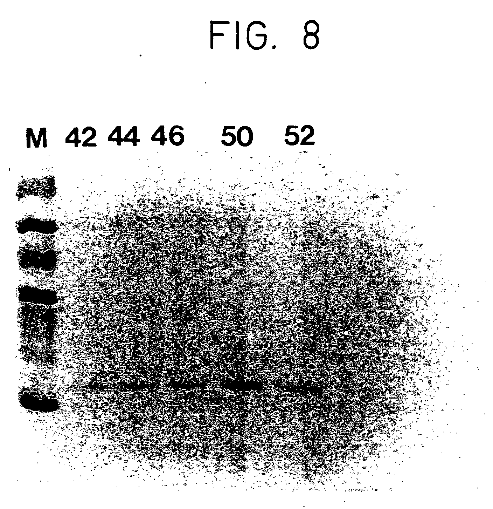

After elution through Sephadex G-75 and CM-cellulose columns and electophoretic analysis on 12.5 PAGE-SDS it is possible to observe the presence of a unique band corresponding to a molecular weight of 18 KDa (Figure 8). By re-eluting the sample through a G-75 column, suitably calibrated with standard proteins, again in native conditions, a band with the same molecular weight can be observed.

According to above a phlogosis marker, detectable in a unique 18 KDa band and present in high amount considering the starting volume, constituted by an ECP form having different electric charge and molecular weight in comparison to the serum form, was obtained.

### Production of Antibodies

Thus obtained ECP form is in such amount and purity conditions that it can be used, according to standard techniques, for the production of specific monoclonal and/or polyclonal antibodies. Thus obtained antibodies are used for a detection system highly sensitive to the ECP present in biological fluids, both according to the ELISA method and other immunoassay methods.

### BIBLIOGRAPHY

1) Gleich GJ. The eosinophil and bronchial asthma: current understanding. J Allergy Clin Immunol 1990; 85: 422-36.
2) O'Sallivan S, Kumlin M. Eosinophil markers in childhood asthma. Clin Exp Allergy 1999; 29: 1454-56.
3) Zimmerman B, Lanner A, Enander 1, Zimmerman RS, Peterson CGB, Ahlstedt S. Total blood eosinophils serum eosinophil cationic protein and eosinophil protein X in childhood asthma: relation to disease status and therapy. Clin Exp Allergy 1993- 23: 564-70.
4) Robinson DS, Assotifi B, Durliam SR, Kay AB. Eosinophil cationic protein (ECP) and eosinophil protein X (EPX) concentration in serum and bronchial lavage fluid in asthma. Effect of prednisolone treatment. Clin Exp Allergy 1995; 25: 1118-27.
5) Linder A, Venge P, Deuschl H. Eosinophil cationic protein and myeloperoxldase in nasal secretion as a marker of infiammation in allergic rhinitis. Allergy 1988; 42: 583-90.
6) Bisgaard H, Grónborg H, Mygind N, DahI R, Lindqvist N, Venge P. Allergen-induced increase of eosinophil cationic protein in nasal lavage fluid: Effect of the glucocorticold budesonide. J Allergy Clin Immunol 1990; 85: 891-5.
7) Konno A. Yamakoshi T, Terada N, Fujlta Y. Mode of action of topical steroid on immediate phase reaction after antigen challenge and non specific nasal hyperreactivity in nasal allergy. Int Arch Allergy Immunol 1994; 103: 79-87.
8) Motojima S, Akutsu 1, Makino S, Takatsu K. Clinical significance of measuring levels of sputum and serum ECP in bronchial asthma. Allergy 1993- 48- 98-106.
9) Sorva R, Metso T, Turpelnen M, Juntunen-Backman K, Bjorksten F, Haalitela T. Eosinophil cationic protein in induced sputum as a marker of inflammation in asthmatic children. Pediatric allergy and immunology 1997; 8: 45-50.
10) Fujisawa T, Terada A, Atsuda J, Iguchi K, Kamiya H, Sakurai M. Clinical utility of serum levels of eosinophil cationic protein (ECP) for monitoring and predicting clinical course in childhood asthma. Clin Exp Allergy 1998; 28: 19-25.
11) Ferguson AC, Vaughan R, Brown H, Curtis C. Evaluation of serum eosinophil cationic protein as a marker of disease activity in chronic asthma. J Allergy Clin Immunol 1995; 95: 23-8.
12) Schmekel B, Blom-Bvìlow B, Hornblad Y, Laltinen LA, Linden M, Venge P. Granulocytes and their secretory products, myeloperoxldase and eosinophil cationic protein, in bronchoalveolar lavage fluids from two lung lobes in normal subjects. Eur Respir J 1991; 4: 867-71.
13) Fahy JV, Liu J, Wong H, Boushey HA. Cellular and biochemical analysis of induced sputum from asthmatic and from healthy subiects. Am Rev Respir Dis 1993, 147: 1126-31.
14) Gleich GJ, Loegering DA, Bell NW, Checkel JL, Ackerman SJ, Mckeand J. Biochemical and functional similarities between human eosinophil derivied neurotoxin and eosinophil cationic protein: homology with ribonuclease. Proc Natl Acad Sci. USA 1986; 83: 3146-50.
15) Christer G B Peterson, Hans Jörnvall, Per Venge. Purification and characterization of eosinophil cationic protein from normal human eosinophils. Eur J Haematol 1988, 40: 415-23.
16) Olsson 1, Venge P. Cationic proteins of human granulocytes. I. Isolation of the cationic proteins of the granules of leukaemia myelold cells. Scand J Haematol 1972; 9: 204-14.
17) Olsson I, Venge P. Cationic proteins of human granulocytes. II. Separation of the cationic proteins of the granules of leukemic myeloid cells. Blood 1974; 44: 235-46.
18) Venge P, Byström J, Carlson M, Håkansson L, Karawacjzyk M, Peterson C, Seveus L, Trulson A. Eosinophil cationic protein (ECP): molecular and biological properties and the use of ECP as a marker of eosinophil activation in disease. Clin Exp Allergy 1999; 29: 1172-86.
19) Fredens K, Dahl R, Venge P. Gordon phenomenon induced by the eosinophil cationic protein and eosinophil protein X. Allergy Clin Immunol 1982, 70: 361-6.
20) Fujisawa T, Abu-Ghazaleh R, Kita H, Sanderson CJ, Gleich GJ Regulatory effect of cytokines on eosinophil degranulation. J Immunol 1990, 144: 642-6.
21) Carlson M, Peterson C, Venge P. The influence of IL-3, IL-5 and GM-CSF on normal human eosinophil and neutrophil C3b-induced degranulation. Allergy 1993; 48: 437-42.
22) Tai P-C, Spry CJF, Petterson C, Venge P, Olsson I. Monoclonal antibodies distinguish between storage and secreted forms of eosinophil cationic protein. Nature 1984; 309: 182-4.
23) Jonas S Erjefalt, Morgan Andersson, Lennart Greiff, Magnus Korsgren, Mariuz Gizycki, Peter K Jeffery, Carl G A Persson. Cytolysis and piecemeal degranulation as distinct modes of activation of airway mucosal eosinophils. J Allergy Clin Immunol 1998; 102: 286-94.
24) Dvorak AM, Furitso T, Letourneau L, Ishizaka T, Ackerman L. Mature eosinophils stimulated to develop in human cord blood mononuclear cell Cultures with human recombinant interleukin-5. I. Piecemeal degranulation of speciflc granules and distribution of Chacot-Leiden crystal protein. Am J Pathol 1997, 138: 69-82.
25) Weller PF, Dvorak A M. Human eosinophils: development, maturation, and functional morphology. In: Busse WW, Holgate ST, editors. Asthma and rhinitis. Boston: Blackwell; 1994. p. 255-74.
26) Persson CGA, Erjefält JS. Eosinophil lysis and free granules: an in vivo paradigm for cell activation and drug development. Trend Pharmacol Sci 1997; 18: 117-23.
27) Erjefält JS, Sundler F, Persson CGA. Eosinophils, neutrophils, and venular gaps in the airway mucosa at epithelial removal-restitution. Am J Respir Crit Care Med 1996, 153: 1666-74.
28) Erjefält JS, Korsgren M, Nilsson MC, Sundler F, Persson CGA. Association between inflammation and epithelial damage-restitution processes in allergic airways in vivo. Clin Exp Allergy 1997; 27: 1345-6.
29) Sensi LG, Seri A, Siracusa A, Pertici L, Marcucci F. Allergic rhinitis in children: effects of flunisolide and disodium cromoglycate on nasal eosinophil cationic protein. Clin Exp Allergy 1997; 27- 270-6.

## Claims

1. Process for the preparation of ECP from nasal mucosa of a subject, including the steps of:
- submitting the subject to a provocation by means of an allergen suitable to induce the allergic phlogosis;
- withdrawing after 24 hours from said subject nasal mucus and putting the same in the presence of a reducing agent;
- submitting said mucus to agitation and centrifugation in order to obtain a supernatant;
- dissolving in said supernatant a chelating agent and concentrating the same from 5 to 30 times to obtain a concentrated sample;
- submitting the concentrated sample to separation processes by molecular weight and/or ionic charge.

2. Process for the preparation of ECP from nasal mucosa of a subject according to claim 1 wherein the allergen suitable to induce the phlogosis is *Dermathophagoides pteronisinus.*

3. Process for the preparation of ECP from nasal mucosa of a subject according to any one of the preceding claims wherein the reducing agent is dithiothreitol.

4. Process for the preparation of ECP from nasal mucosa of a subject according to any one of the preceding claims wherein the chelating agent is EDTA.

5. Process for the preparation of ECP from nasal mucosa of a subject according to any one of the preceding claims wherein the processes of separation by molecular weight include filtration through a Sephadex G-75 column.

6. Process for the preparation of ECP from nasal mucosa of a subject according to any one of the preceding claims wherein the processes of separation by ionic charge include ion exchange chromatography through a CM-cellulose column.

7. ECP protein obtainable according to the method of any one of the preceding claims from nasal mucus of subjects subjected to an allergen provocation.

8. Method for monitoring the phlogosis from allergic reaction of a subject, including the steps of:
- incubating a biological fluid from the subject with an antibody obtained by immunization using the ECP protein according to claim 7;
- detecting the antigen-antibody reaction.

9. Method according to claim 8 wherein the detection of the antigen-antibody reaction is carried out using an additional antibody conjugated to a marker.
